# EUROPEAN PATENT APPLICATION

(11) **EP 2 286 777 A1**
(43) Date of publication of application: **23.02.2011**
(21) Application number: 09746434.1
(22) Date of filing: 23.03.2009
(51) Int. Cl.: A61F 13/49, A61F 13/56, A61F 13/66

(54) **WEARING ARTICLE**

(30) Priority: 15.05.2008 JP 2008128929
(71) Applicant: Uni-charm Corporation, Ehime 799-0111 (JP)
(72) Inventor: KINOSHITA, Akiyoshi, Kanonji-shi Kagawa 769-1602 (JP); AOYAGI, Natsuko, Kanonji-shi Kagawa 769-1602 (JP); TANAKA, Kayoko, Kanonji-shi Kagawa 769-1602 (JP); KENMOCHI, Yasuhiko, Kanonji-shi Kagawa 769-1602 (JP)
(74) Representative: Murgatroyd, Susan Elizabeth
(86) International application number: PCT/JP2009/055602
(87) International publication number: WO 2009/139227

(57) **Abstract**

The present invention aims to provide a wearing article improved so that the wearing article can be smoothly put on the wearer's body even when the wearer is in the chair. Of front and rear waist regions 13, 14, at least the rear waist region 14 is elasticized, fastening means comprising a pair of first fastening elements extending on respective outer surfaces of first lateral zones of the front waist region in a longitudinal direction and a pair of second fastening elements adapted to be engaged with the associated the first fastening elements and extending on respective inner surfaces of second lateral zones of the rear waist region in the longitudinal direction, and assumed that after the first fastening element has been engaged with the associated second fastening element on one side of the wearing article, the front and rear waist regions are pulled toward the other side and, while at least the rear waist region is held in its stretched state and the first fastening element is tried to be engaged with the associated second fastening element, the fastening means exhibit sufficiently high fastening strength against shearing force to prevent the first and second fastening elements on the one side as well as on the other side from unintentionally being disengaged from each other.

## Description

### TECHNICAL FIELD

The present invention relates to pant-type wearing articles such as disposable pant-type diapers or toilet-training pants and more particularly to pant-type wearing articles having respective pairs of lateral zones of front and rear waist regions adapted to be detachably engaged with each other.

### RELATED ART

The disposable diaper in which the front and rear waist regions are adapted to be detachably connected with each other along respective pairs of lateral zones thereof has conventionally known. For example, the diaper disclosed in PATENT DOCUMENT 1 comprises a liquid-absorbent chassis and fastening means wherein the chassis comprises a front waist region, a rear waist region and a crotch region and the fastening means respectively includes hook elements and loop elements.

### [PATENT DOCUMENT 1] JP 2008-12115 A

### DISCLOSURE OF THE INVENTION

### PROBLEM TO BE SOLVED BY THE INVENTION

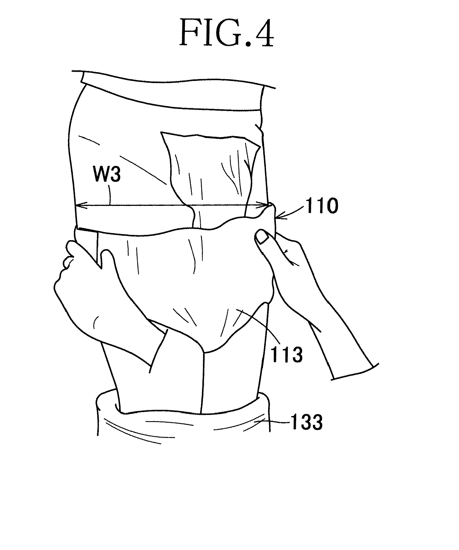

In the case of the diaper 110 disposed in PATENT DOCUMENT 1, the front waist region 113 and the rear waist region may be connected with each other along the respective lateral zones via the fastening means to shape the diaper 110 into the pant. The diaper 110 shaped into the pant can be smoothly put on the wearer's body even while standing the wearer up without taking off his or her trousers (See Fig. 4).

However, if the wearer's garment 133 such as trousers are pulled down while standing the wearer up, a posture of the wearer might become unstable, particularly in the case of the aged wearer, resulting in getting off balance, staggering about and even tipping over. To avoid such situation, a helper often puts the aged wearer in a chair to put the diaper on the wearer's body in such posture. Assumed that, during this, the wearer is in the chair with his or her garment 133 such as trousers around the ankles, the garment 133 wedged between the ankles force the ankles to be spaced from each other. Consequentially, the wearer's thighs are correspondingly spaced from each other so that a width dimension as measured between respective outer sides of the thighs should be larger than a dimension of the wearer's waist. If the diaper 110 is guided between the wearer's thighs and the front and rear waist regions of the diaper are connected to each other in order to shape the diaper 110 into the pant, the diaper 110 will be pulled outward in the transverse direction beyond a supposed width dimension (width dimension of the waist as measured in the transverse direction) . As a result, the engagement between the front and rear waist regions might be released in the course of putting the diaper 110 on the wearer's body.

In view of the problem as has been described just above, it is a principal object of the present invention to provide a wearing article improved so that the wearing article can be smoothly put on the wearer's body even when the wearer is in the chair.

### MEASURE TO SOLVE THE PROBLEM

The object set forth above is achieved, according to the present invention, by an improvement in the wearing article having a longitudinal direction and a transverse direction and comprising a liquid-absorbent chassis and fastening means wherein the liquid-absorbent chassis includes a front waist region, a rear waist region and a crotch region extending between these front and rear waist regions and the fastening means serve to connect a pair of opposite lateral zones of the front waist region referred to hereinafter as first lateral zones with a pair of opposite lateral zones of the rear waist region referred to hereinafter as second lateral zones in a detachable fashion so as to define a waist-opening and a pair of leg-openings.

The improvement according to the present invention is characterized in that at least the rear waist region of the front waist region and the rear waist region is elasticized, the fastening means comprise a pair of first fastening elements extending on respective outer surfaces of the first lateral zones of the front waist region in the longitudinal direction and a pair of second fastening elements adapted to be associated the first fastening elements and extending on respective inner surfaces of the second lateral zones of the rear waist region in the longitudinal direction, and assumed that after the first fastening element has been engaged with associated the second fastening element on one side of the wearing article, the front and rear waist regions are pulled toward the other side and at least the rear waist region is held in its stretched state and the first fastening element is tried to be engaged with associated the second fastening element, the fastening means exhibit sufficiently high fastening strength against shearing force to prevent the sufficiently high to prevent the first and second fastening elements on the one side as well as on the other side from unintentionally being disengaged from each other.

In addition to the representative embodiment as has been described just above, the present invention may be implemented in preferred embodiments as follow:
(1) The fastening means are provided in the form of mechanical fasteners, wherein the first fastening elements and the second fastening elements are implemented in a form of hook elements and loop elements adapted to be engaged with associated the hook elements.
(2) The fastening means are implemented in a form of mechanical fasteners and shearing stress generated by engagement between the first fastening elements and the second fastening elements is 5N/cm² or higher and fastening strength against shearing force exhibited by the fastening means as a whole is 60N or higher.
(3) The second fastening elements extending on respective inner surfaces of the second lateral zones of the rear waist region and the first fastening elements are attached to respective outer surfaces of base members extending outward from the first lateral zones.
(4) The first fastening element is divided into upper halves and lower halves spaced from and opposed to each other in the longitudinal direction.

### EFFECT OF THE INVENTION

According to the present invention, the fastening means provided in the transversely opposite lateral zones of the diaper have a sufficiently high level of fastening strength against shearing force to overcome the problem such that, even when the diaper is put on the wearer's body sitting in the chair by connecting the respective lateral zones of the front and rear waist regions on one side of the diaper with each other vua the associated fastening means and then by connecting the respective lateral zones at the other side of the diaper via the fastening means provided on the other side of the diaper while at least the rear waist region is stretched toward the other side of the diaper, the fastening means on one side and/or on the other side might be disengaged in this course of putting the diaper on the wearer's body.

### BRIEF DESCRIPTION OF THE DRAWINGS

Fig. 1 is a perspective view of the disposable diaper.
Fig. 2 is a partially cutaway plan view of the flatly developed disposable diaper.
Fig. 3 is a diagram illustrating a sequence from A to B in which the diaper is put on the wearer sitting in a chair.
Fig. 4 is a diagram illustrating a manner in which the diaper of prior art is put on the wearer in upright posture.

### IDENTIFICATION OF REFERENCE NUMERALS USED IN THE DRAWINGS

- 10: disposable diaper (wearing article)
- 11: chassis
- 12a, 12b: fastening means
- 13: front waist region
- 13a, 13b: first pair of lateral zones
- 14: rear waist region
- 14a, 14b: second pair of lateral zones
- 15: crotch region
- 16: waist-opening
- 17: leg-opening
- 25, 26: first pair of fastening elements
- 25a, 26a: upper halves
- 25b, 26b: lower halves
- 27, 28: second pair of fastening elements
- 29a, 29b: base members
- 30: unoccupied space
- X: transverse direction
- Y: longitudinal direction

### DETAILED DESCRIPTION OF THE PREFERRED EMBODIMENTS

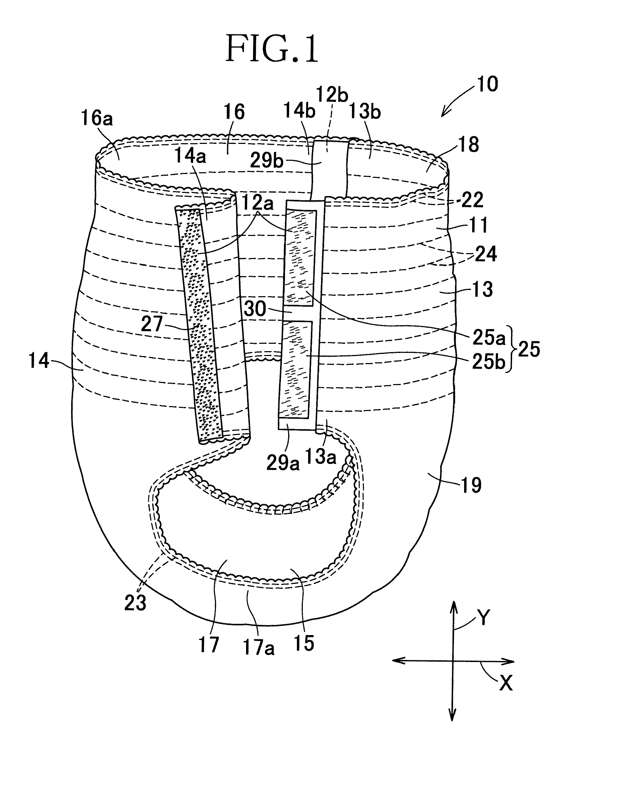

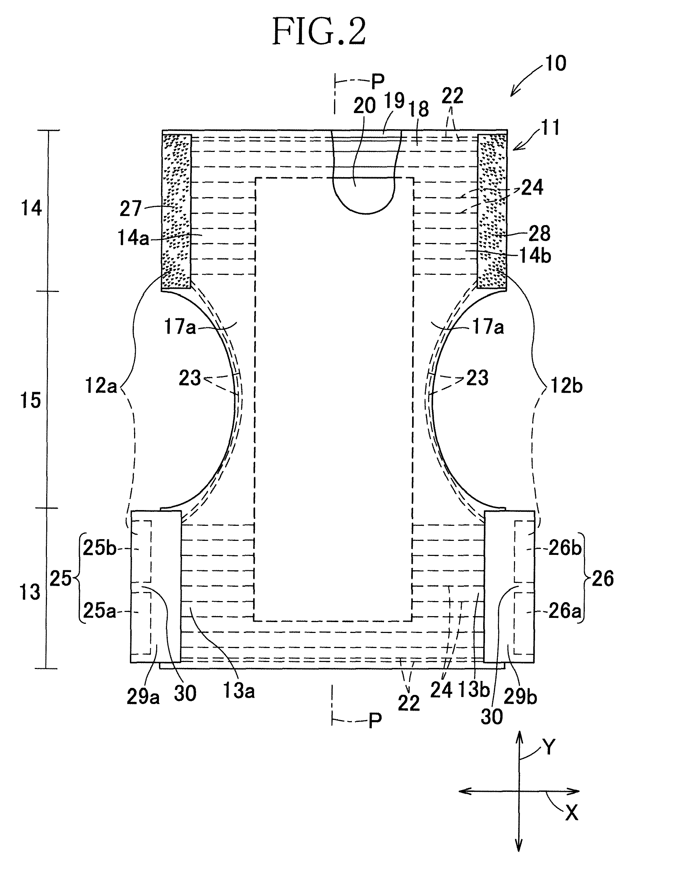

Figs. 1 through 3 illustrate a disposable diaper 10 for adult as a typical embodiment of the present invention and details of the invention will be more fully understood from the description of this diaper for adult given hereunder in reference with these Figs. 1 through 3. Fig. 1 is a perspective view of the diaper 10 with a pair of fastening means 12a disengaged from each other and Fig. 2 is a partially cutaway plan view of the diaper 10 flatly developed in a transverse direction X as well as in a longitudinal direction Y of the diaper 10 after both pairs of the fastening means 12a, 12b have been disengaged one from another.

As will be apparent from Figs. 1 and 2, the diaper 10 comprises a liquid-absorbent chassis 11 and the fastening means 12a, 12b and is configured from a front waist region 13, a rear waist region 14 and a crotch region 15 extending between these waist regions 13, 14.

The chassis 11 comprises an inner sheet 18 formed by liquid-pervious nonwoven fabric, an outer sheet 19 formed by hydrophobic fibrous nonwoven fabric, moisture-pervious plastic film or a laminate thereof, and a liquid-absorbent core 20 sandwiched between the inner and outer sheets 18, 19 wherein the chassis 11 is curved inward so as to be constricted in the crotch region 15 and to have a concave shape curved inwardly as a whole.

In the front and rear waist regions 13, 14, a plurality of strand-like elastic elements are sandwiched between the inner and outer sheets 18, 19 and attached under tension, i.e., in stretchable/contractible manner by hot melt adhesive (not shown). More specifically, these strand-like elastic elements comprise a plurality of first waist elastic elements 22 extending in the transverse direction X along a peripheral edge 16a of the waist-opening 16, a plurality of leg elastic elements 23 extending along respective peripheral edges 17a of the leg-openings 17, and a plurality of second waist elastic elements 24 extending in the transverse direction X provided between the peripheral edge 16a of the waist-opening 16 and the respective peripheral edges 17a of the leg elastic elements 17.

These elastic elements 22, 23, 24 are made of natural or synthetic rubber. While these elastic elements are strand-like in the illustrated embodiment, it is possible to use tape-like elastic elements. It is also possible to use elasticized sheets as inner and outer sheets instead of using the elastic elements 22, 23, 24 provided in the front and rear waist regions 13, 14.

The core 20 is formed typically by a mixture of fluff pulp and superabsorbent polymer particles (SAP) and preferably wrapped with a liquid-dispersant shape retaining sheet (not shown) to prevent the core 20 from unacceptable deformation and at the same time to prevent potential falling off of SAP.

The front waist region 13 and the rear waist region 14 respectively have a first pair of lateral zones 13a, 13b and a second pair of lateral zones 14a, 14b, both pairs being symmetric about a longitudinal axis P of the diaper 10 and extending in the longitudinal direction Y. The first pair of lateral zones 13a, 13b and the second pair of lateral zones 14a, 14b may be connected to each other by the fastening means 12a, 12b to put the diaper 10 on the wearer's body. Upon connection of these first and second pairs of lateral zones 13a, 13b, 14a, 14b in this manner, the waist-opening 16 and a pair of the leg-openings 17 are formed (See Fig. 1).

The fastening means 12a, 12b comprise a first pair of fastening elements 25, 26 and a second pair of fastening elements 27, 28 respectively adapted to be engaged with the first pair of fastening elements 25, 26. The first pair of fastening elements 25, 26 are bonded to respective outer surfaces of base members (i.e., ear-like members) 29a, 29b extending outward from the first lateral zones 13a, 13b in the transverse direction X and the second pair of fastening elements 27, 28 are attached to respective inner surfaces of the second lateral zones 14a, 14b, by adhesive, thermal or ultrasonic bonding technique.

The base members 29a, 29b are formed by fibrous sheets such as nonwoven fabric, woven fabric or net fabric and respectively have substantially same length as the longitudinal dimension of the first lateral zones 13a, 13b. These base members 29a, 29b are attached to respective inner surfaces of the first lateral zones 13a, 13b by adhesive or thermal or ultrasonic bonding technique of well known art so as to extend outward from the first lateral zones 13a, 13b in the transverse direction X. The base members 29a, 29b are attached to the associated first lateral zones 13a, 13b without overlapping the elastic members 22, 23, 24. In this way, the base members 29a, 29b would not be affected by contraction of these elastic elements 22, 23, 24 and consequently the expected fastening function thereof would not be deteriorated.

The first fastening elements 25, 26 are bonded to the respective outer surfaces of the base members 29a, 29b by adhesive or thermal or ultrasonic bonding technique of well known art so that the first fastening elements 25, 26 respectively comprise upper halves 25a, 26a and lower halves 25b, 26b spaced from and opposed to one another in the longitudinal direction Y, leaving therebetween unoccupied spaces 30. The unoccupied spaces 30 defined between the upper halves 25a, 26a and the lower halves 25b, 26b may be conveniently utilized when it is desired to put the diaper 10 on the wearer's body. Specifically, the upper halves 25a, 26a may be engaged with the associated second fastening elements 27, 28 for positioning and thereafter the lower halves 25b, 26b may be engaged with the second fastening elements 27, 28 to achieve desired handling for engagement. In this way, the handling for engagement is facilitated even in the case of the diaper exclusively used for adult and having the fastening means 12a, 12b larger than those in the diaper for baby. Also when the used diaper 10 is taken off from the wearer's body, handling for disengagement can be carried out more easily than for the diaper of prior art. Specifically, the helper's fingers may be inserted into respective gaps defined by the unoccupied spaces 30 between the first fastening elements 25, 26 and the second fastening elements 27, 28 engaged together and then the helper's fingers may be pressed against the unoccupied spaces 30 so as to disengage the first fastening elements 25, 26 and the second fastening elements 27, 28 one from another. It should be appreciated here that it is not essential to divide the first fastening elements 25, 26 into the upper and lower halves but it is also possible to use the elements 25, 26 which continuously extend in the longitudinal direction Y.

The first fastening elements 25, 26 may be appropriately dimensioned depending on a particular size of the diaper and, in the case of the normal diaper 10 for adult, each of the first fastening elements 25, 26 preferably has a width in a range of 1. 0 to 1. 7 cm and a length in a range of 6.0 to 20.0 cm to assure a sufficiently high fastening strength of the fastening means 12a, 12b against shearing force. However, such dimensions are not limitations but may be appropriately varied depending on factors such as the size of the diaper. The term "fastening strength against shearing force" used herein refers to a strength of the fastening means 12a, 12b against the shearing force exerted on planes along which the both fastening means 12a, 12b are respectively held in states of engagement when one of the fastening means 12a, 12b having been brought into engagement and the front and rear waist regions 13, 14 are pulled toward the other of the fastening means 12a, 12b to bring the other of the fastening means 12a, 12b into engagement.

The second fastening elements 27, 28 extend substantially over the entire second lateral zones 14a, 14b in the longitudinal direction Y and attached to the respective inner surfaces of these second lateral zones 14a, 14b by adhesive or thermal or ultrasonic bonding technique of well known art. The second fastening elements 27, 28 extending substantially over the entire second lateral zones 14a, 14b in the longitudinal direction Y in this manner assure sufficient area of engagement even if the first fastening elements 25, 26 are somewhat displaced from proper positions in engagement with the associated second fastening elements 27, 28.

The fastening means 12a, 12b may be implemented in the form of mechanical fasteners wherein the first fastening elements 25, 26 may be implemented in the form of hook elements serving as the male fasteners constituting the respective mechanical fasteners and the second fastening elements 27, 28 may be implemented in the form of loop elements serving as the female fasteners constituting the respective mechanical fasteners adapted to be detachably engaged with the associated hook elements. While the first fastening elements 25, 26 are the hook elements and the second fastening elements 27, 28 are loop elements according to the illustrated embodiment, it is possible to provide the first fastening elements 25, 26 as the loop elements and to provide the second fastening elements 27, 28 as the hook elements. Alternatively, it is also possible to eliminate the second fastening elements 27, 28 at all and to attach the first fastening elements 25, 26 provided in the form of the hook elements directly to the respective inner surfaces of the second lateral zones 14a, 14b so that the first fastening elements 25, 26 provided in the form of the hook elements may be engaged with a fibrous sheet such as fibrous nonwoven fabric forming the inner sheet 18.

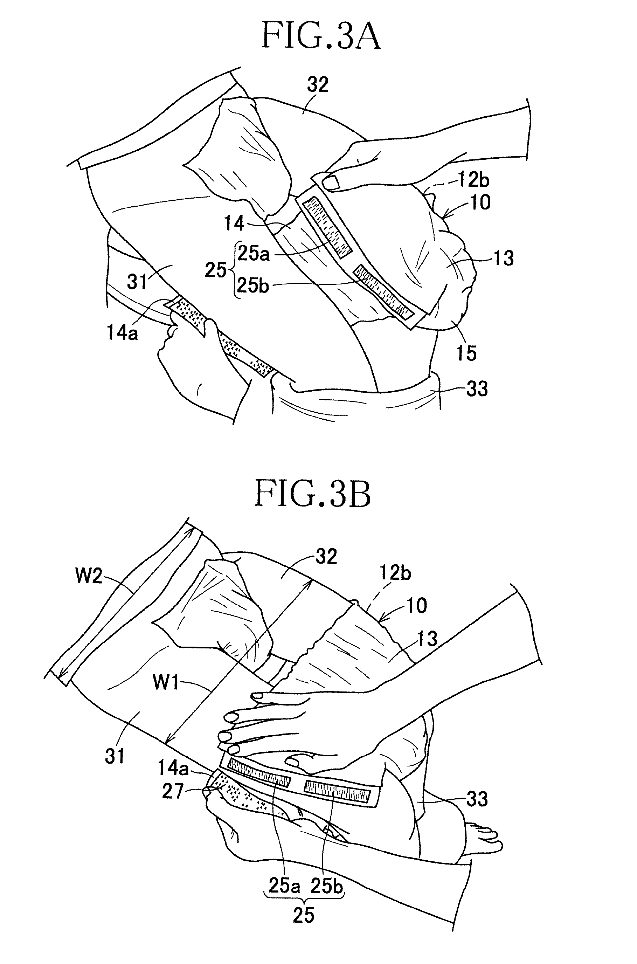

Fig. 3 is a diagram illustrating a sequence from A to B in which the helper puts the diaper 10 on the wearer's body sitting in a chair and Fig. 4 is a diagram illustrating a manner in which the diaper 110 of prior art is put on the wearer in upright posture. Expressions "right-hand side" and "left-hand side" used herein should be understood to be designated with respect to the helper.

Now the sequence in which the helper puts the diaper 10 on the wearer will be described. Referring to Fig. 3A first, the crotch region 15 is positioned between the wearer's thighs 31, 32, the front waist region 13 is placed on respective upper sides of the thighs 31, 32 while the rear waist region 14 is placed on respective lower sides of the thighs 31, 32, then the first lateral zone 13b is engaged with the second lateral zone 14b, and thereafter the front waist region 13 is held in the vicinity of upper end thereof with the right hand and pulled leftward while the second lateral zone 14a of the rear waist region 14 underlying the thigh 31 is held with the left hand and pulled leftward. Referring now to Fig. 3B, while holding the front waist region 13 as having been pulled leftward and pressing it against the upper side of the wearer's thigh 31 to hold it thereon, the second lateral zone 14a is pulled up from under the thigh 31 until the second lateral zone 14a is put flat together with the first lateral zone 13a on the lateral side of the thigh 31, and finally the upper half 25a and the lower half 25b of the first fastening element 25 are successively engaged with the second fastening element 27 to shape the pant 10 into pant.

Assumed that, during this, the wearer is in the chair with his or her garment 33 such as trousers around the ankles, the garment 33 wedged between the ankles force the ankles to be spaced from each other. Consequentially, the wearer's thighs 31, 32 are correspondingly spaced from each other so that a width dimension W1 as measured between respective outermost sides of the wearer's thighs should be larger than a width dimension W2 the wearer's waist. As will be apparent from Fig. 4, the width dimension W1 will be larger than a width dimension W3 as measured between the respective outermost sides when the diaper 110 is put on the wearer in upright posture.

In other words, tensile stress in the transverse direction X is exerted on the diaper 10 its entirety and the diaper 10 is stretched in the transverse direction X beyond an assumed width (i.e., the width dimension W2 of the waist). In consequence, the plane of the fastening means 12b along which the first fastening element 26 and the second fastening element 28 are first engaged is exposed to shearing force higher than that exerted on the corresponding plane when the diaper 10 is put on the wearer's body in upright posture. Therefore, if fastening strength of the fastening means 12a, 12b is relatively low, the first and second fastening elements 26, 28 of the first fastening means 12b having been first engaged with each other might be disengaged from each other when it is tried to engage the first and second fastening elements 25, 27 of the fastening means 12a with each other, eventually making it difficult to perform the handling of putting the diaper 10 on the wearer's body smoothly.

To overcome such anxiety, the fastening means 12a, 12b according to the present invention are provided with fastening strength against shearing force sufficiently high to avoid the above-mentioned anxiety that the fastening effect might be lost in the course of putting the diaper 10 on the wearer's body.

To meet such requirements, shear stress generated as the first fastening element is engaged with the associated second fastening means is preferably in a range of 5N/cm² or higher and the fastening means 12a, 12b preferably exhibit fastening strength against shearing force of 60N or higher. If the fastening strength against shearing force of the fastening means 12a, 12b is less than 60N, the fastening means 12b brought in engagement first or/and the fastening means 12 brought in engagement thereafter might be unintentionally disengaged when the diaper 10 is put on the wearer's body sitting in the chair.

The shearing stress of the fastening means 12a, 12b may be measured by a method as follows:

First, from the hook element and the loop element used for the first fastening elements 25, 26 and the second fastening elements 27, 28, respectively, a test piece of the hook element having a width of 25 mm and a length of 20 mm and a test piece of the loop element having a width of 50 mm and a length of 50 mm are cut off. The test piece of the hook element is placed substantially on a central zone of the test piece of the loop element in a manner that the hook element may face the loop element. Now a pressure roller having a weight of 2 kg is reciprocated only once on this assembly to bring these two elements into engagement. The surface of the loop element not in engagement with the hook element (hook backing) is immobilized on stainless plate by double-faced adhesive tape while the surface of the hook element not in engagement with the loop element (loop backing) is immobilized on inelastic paper sheet for tensile test by pressure-sensitive adhesive tape. One end of the stainless plate and an end of the paper sheet for tensile test opposed to the end of the stainless plate are held by chucks of a tensile tester. Now the stainless plate and the paper sheet for tensile test held in the chucks are pulled in opposite directions, i.e. , in the shearing direction for the plane in which the hook element is engaged with the loop element at a moving rate of 10 mm/min. A value at a moment of disengagement (i.e., the maximum load value) is determined as the strength against shearing force.

As has previously been described, the front and rear waist regions 13, 14 are provided with the first and second waist elastic members 22, 24 to elasticize the front and rear waist regions 13, 14, allowing the front and rear waist regions 13, 14 to be rather easily pulled in the transverse direction when the diaper 10 is put on the wearer's body in sitting posture. In order to facilitate operation of putting the diaper 10 on the wearer's body, it is possible to elasticize the rear waist region 14 alone and/or to dimension the rear waist region 14 to be larger than the front waist region 13. If the rear waist region 14 alone is elasticized and the rear waist region 14 is dimensioned to be larger than the front waist region 13, shearing force exerted on the previously brought into engagement can be effectively alleviated in the course of putting the diaper 10 on the wearer's body because it is unnecessary to stretch the front waist region 13.

In the course of putting the diaper 10 on the wearer's body, the first and second waist elastic elements 22, 24 preferably exhibit a stretch ratio in a range of 2.0 to 4.0 for sufficient elongation of the front and rear waist region 13, 14. Furthermore, in order to facilitate the diaper 10 to be put on the wearer's body, the first and second waist elastic elements 22, 24 preferably exhibit a higher stretch ratio as well as a lower stretch stress in comparison to those of the waist elastic elements usually used in the conventional diaper of this type. The stretch ratio as well as the stretch stress set in this manner for the diaper according to the invention allows the front and rear waist regions 13, 14 to be adequately stretched, on one hand, and allows the shearing force exerted on the fastening means 12a, 12b under contraction of these elastic elements to be effectively alleviated. While it is known for the diaper of this type, the first waist elastic elements 22 extending along the peripheral edge 16a of the waist-opening 16 preferably exhibit the stretch ratio as well as the stretch stress higher than those of the second waist elastic elements 24 provided principally for the purpose of improved fitness to the wearer's body.

While the diaper 10 according to the illustrated embodiment comprises the chassis 11 including the core 20 principally serving for liquid-absorption sandwiched between the inner and outer sheets 18, 19, an alternative construction is also possible such that the diaper 10 comprises the chassis formed by a sheet member and a pad-like liquid-absorbent structure including the core 20 prepared separately of the chassis 11. In this case, the liquid-absorbent structure may be attached to the inner surface of the chassis 11 only along limited portions of its front and rear ends and transversely opposite side edges to avoid a possibility that the presence of the core 20 having a relatively high stiffness might obstruct smooth stretching as well as contraction of the second waist elastic elements 24. Consequentially, the front and rear waist regions 13, 14 can be smoothly stretched in the course of putting the diaper 10 on the wearer's body.

## Claims

1. A wearing article having a longitudinal direction and a transverse direction and comprising a liquid-absorbent chassis and fastening means wherein said liquid-absorbent chassis including a front waist region, a rear waist region and a crotch region extending between said front and rear waist regions and said fastening means serve to connect a pair of first lateral zones of said front waist region with a pair of second lateral zones of said rear waist region in a detachable fashion so as to define a waist-opening and a pair of leg-openings, said wearing article being **characterized in that**:
at least said rear waist region of said front waist region and said rear waist region is elasticized;
said fastening means comprise a pair of first fastening elements extending on respective outer surfaces of said first lateral zones of said front waist region in said longitudinal direction and a pair of second fastening elements adapted to be associated said first fastening elements and extending on respective inner surfaces of said second lateral zones of said rear waist region in said longitudinal direction; and
assumed that after said first fastening element has been engaged with associated said second fastening element on one side of said wearing article, said front and rear waist regions are pulled toward other side and, while at least said rear waist region is held in its stretched state and said first fastening element is tried to be engaged with associated said second fastening element, said fastening means exhibit sufficiently high fastening strength against shearing force to prevent said first and second fastening elements on said one side as well as on said other side from unintentionally being disengaged from each other.

2. The wearing article as defined by Claim 1, wherein said fastening means are provided in a form of mechanical fasteners, wherein said first fastening elements and said second fastening elements are implemented in a form of hook elements and loop elements adapted to be engaged with associated said hook elements.

3. The wearing article as defined by Claim 1 or 2, wherein said fastening means are implemented in a form of mechanical fasteners and shearing stress generated by engagement between said first fastening elements and said second fastening elements is 5N/cm² or higher and fastening strength against shearing force exhibited by said fastening means as a whole is 60N or higher.

4. The wearing article as defined by any one of Claims 1 through 3, wherein said second fastening elements extend on respective inner surfaces of said second lateral zones of said rear waist region and said first fastening elements are attached to respective outer surfaces of base members extending outward from said first lateral zones.

5. The wearing article as defined by any one of Claims 1 through 4, wherein said first fastening element is divided into upper halves and lower halves spaced from and opposed to each other in said longitudinal direction.
